# DEMANDE DE BREVET EUROPEEN

(11) **EP 1 894 540 A1**
(43) Date de publication de la demande: **05.03.2008**
(21) Numéro de dépôt: 06119749.7
(22) Date de dépôt: 30.08.2006
(51) Int. Cl.: A61C 1/18, A61B 17/16, F16B 7/20

(54) **Instrument à main à usage dentaire ou chirurgical**

(71) Demandeur: Bien-Air Holding SA, 2504 Bienne (CH)
(72) Inventeur: Maitre, Luc, 2885 Epauvillers (CH)
(74) Mandataire: GLN

(57) **Abrégé**

L'invention concerne un instrument médical à main destiné à faire tourner à grande vitesse un outil amovible, du type comportant un mécanisme à pince pour la fixation de l'outil, un mécanisme moteur pour l'entraînement en rotation du mécanisme à pince et un système d'accouplement (21) du mécanisme à pince avec le mécanisme moteur, ledit système comportant un arbre creux (28) à l'intérieur duquel l'extrémité (20) de l'arbre du mécanisme à pince est capable de coulisser. Selon l'invention, le système d'accouplement (21) et l'extrémité (20) de l'arbre du mécanisme à pince s'assemblent l'un à l'autre au moyen d'une fixation à baïonnette dans laquelle des ergots (26) appartenant à ladite extrémité (20) sont retenus, après rotation, par une butée (30) ménagée à l'intérieur dudit arbre creux (28).

## Description

La présente invention se rapporte au domaine des instruments médicaux. Elle concerne, plus particulièrement, un instrument à main destiné à faire tourner à grande vitesse un outil amovible dont l'extrémité constitue une fraise ou organe analogue utilisable dans les cabinets ou laboratoires dentaires ainsi qu'en microchirurgie.

Un instrument de ce type est décrit, par exemple, dans le document WO 2005/089666. Typiquement, la queue de l'outil est maintenue par une pince disposée à l'intérieur d'un arbre rotatif creux entraîné à son extrémité par un moteur électrique ou un moteur à turbine à air et monté par des roulements dans un fourreau tubulaire fixe. Le serrage et le deserrage de la pince se fait en tournant dans un sens ou l'autre un manchon monté rotatif sur le fourreau.

Un tel instrument est généralement réalisé en trois parties, à savoir un mécanisme à pince avec ses arbres menant et mené pour la fixation de l'outil, un mécanisme moteur pour l'entraînement en rotation du mécanisme à pince et un système d'accouplement du mécanisme à pince avec le mécanisme moteur.

Ces trois composants doivent pouvoir être aisément séparés les uns des autres afin de permettre au même mécanisme moteur d'actionner différents types de mécanisme à pince et d'autoriser le remplacement du système d'accouplement soumis à des sollicitations qui accélèrent son usure.

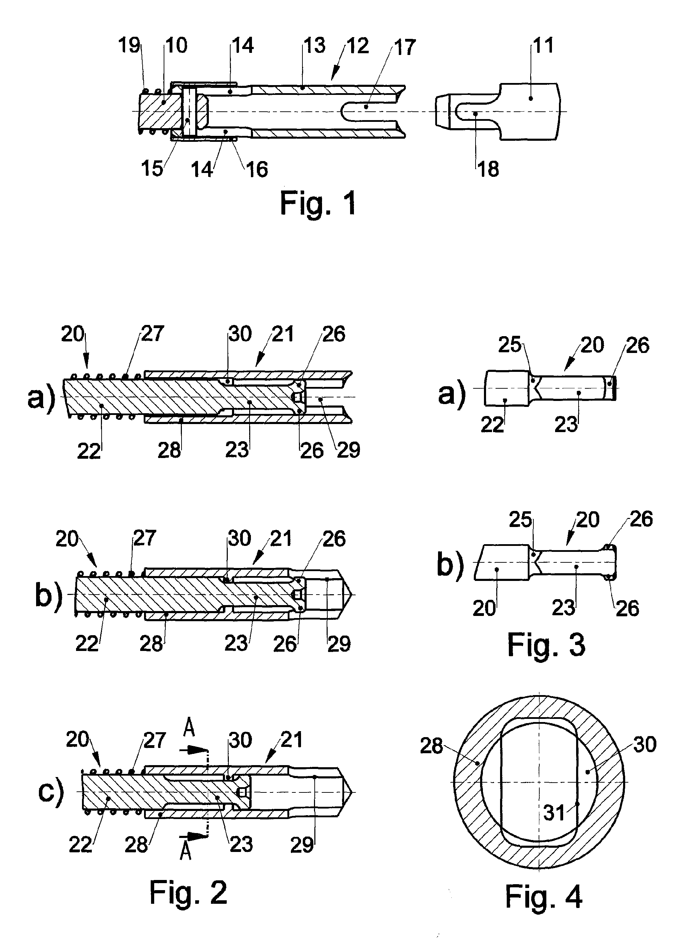

On se référera, tout d'abord, à la figure 1 qui montre, dans une réalisation de l'art antérieur, la manière dont les trois composants sont assemblés. Afin de faciliter la lecture, la présente description utilisera les mots « amont » et « aval » pour désigner respectivement l'extrémité de l'instrument à partir de laquelle l'outil est entraîné en rotation (partie droite du dessin) et son extrémité portant la pointe active de l'outil (partie gauche du dessin). De plus, le dessin ne montre pas les parties amont et aval de l'instrument car celles-ci peuvent être identiques à celles décrites dans le document WO 2005/089666 précité, auquel on pourra se référer pour comprendre la manière dont se fait l'entraînement de l'outil.

Sur la figure 1, on a représenté en 10 l'extrémité amont de l'arbre menant du mécanisme à pince, en 11 l'extrémité aval de l'arbre du mécanisme moteur et en 12 le système d'accouplement selon l'invention.

Celui-ci comporte un arbre creux 13 doté, à son extrémité aval, de deux fenêtres axiales oblongues 14 diamétralement opposées. L'extrémité amont 10 de l'arbre du mécanisme à pince est montée coulissante dans l'extrémité aval de l'arbre 13 et traversée par une goupille de connexion amovible 15 dont la course est limitée axialement par les deux fenêtres 14. Une douille fendue 16, clipsée autour de la portion de l'arbre 13 qui comporte ces fenêtres, assure le maintien en place de la goupille 15. Pour séparer l'arbre menant du mécanisme à pince du système d'accouplement, il est donc nécessaire de déclipser la douille 16 afin de faire apparaître la goupille 15 qui peut donc être retirée et permet ainsi de séparer les arbres 10 et 13.

L'extrémité amont de l'arbre 13 est dotée de deux encoches axiales oblongues 17 diamétralement opposées (une seule apparaissant au dessin) qui sont dimensionnées et conformées de manière à recevoir deux ailettes 18 (une seule apparaissant au dessin) qui terminent l'arbre 11 du mécanisme moteur. Selon une variante de réalisation, le nombre de paires d'encoches diamétralement opposées peut être plus élevé, afin de faciliter l'insertion des ailettes 18.

On notera, enfin, la présence d'un ressort 19 qui est disposé autour de l'arbre 10 et a pour effet de repousser l'arbre 13 vers l'amont. Grâce à ce ressort, même si le praticien n'a pas pris soin d'arrêter le moteur lorsqu'il connecte le mécanisme moteur au système d'accouplement, l'arbre 13 a toujours la possibilité de se rétracter plus ou moins afin de faciliter l'opération

Si un tel mode de connexion du mécanisme moteur au système d'accouplement donne pleine satisfaction, il n'en est pas de même pour la connexion du système d'accouplement à l'arbre menant du mécanisme à pince. En effet, la solution décrite ci-dessus fait appel à une douille assez complexe dont la réalisation n'est pas aisée et dont le montage ou démontage nécessite un ouillage spécial. De plus, cette douille fendue clipsée ne garantit pas une tenue capable de résister à une connexion du système d'accouplement au mécanisme moteur alors que le moteur est toujours en rotation.

La présente invention a notamment pour but de fournir un instrument à main exempt des inconvénients mentionnés ci-dessus.

De façon plus précise, l'invention concerne un instrument à main, du type comportant un mécanisme à pince pour la fixation de l'outil, un mécanisme moteur pour l'entraînement en rotation du mécanisme à pince et un système d'accouplement du mécanisme à pince avec le mécanisme moteur, ledit système comportant un arbre creux à l'intérieur duquel l'extrémité de l'arbre du mécanisme à pince est capable de coulisser. Selon l'invention, le système d'accouplement et l'extrémité de l'arbre du mécanisme à pince s'assemblent l'un à l'autre au moyen d'une fixation à baïonnette dans laquelle des ergots appartenant à ladite extrémité sont retenus, après rotation, par une butée ménagée à l'intérieur dudit arbre creux.

D'autres caractéristiques et avantages de la présente invention ressortiront de la description qui va suivre, faite en regard du dessin annexé dans lequel :
- la figure 2 est une vue partielle en coupe longitudinale de l'instrument selon l'invention dans trois positions caractéristiques montrées en a, b et c;
- les figures 3a et 3b représentent l'extrémité amont de l'arbre du mécanisme à pince, vue sous des angles différant de 90°; et
- la figure 4 est une vue en coupe diamétrale selon AA du système d'accouplement.

Sur la figure 2, on a représenté en 20 l'extrémité amont de l'arbre du mécanisme à pince et en 21 le système d'accouplement de l'extrémité 20 à l'arbre du mécanisme moteur (non représenté mais identique à celui de la figure 1). Selon une caractéristique principale de l'invention, l'arbre du mécanisme à pince et le système d'accouplement s'assemblent l'un à l'autre au moyen d'une fixation à baïonnette dotée d'ergots retenus par rotation.

Comme le montrent aussi les figures 3a et 3b, l'extrémité amont 20 de l'arbre du mécanisme à pince comporte une portion cylindrique 22 suivie d'une portion amincie à section carrée 23. Une saignée circulaire 25 est pratiquée dans la portion amincie 23, à l'endroit de sa liaison avec la portion cylindrique 22. Enfin, la portion amincie 23 se termine par deux ergots 26, diamétralement opposés, qui font partie de la fixation à baïonnette déjà mentionnée.

Il faut aussi noter la présence d'un ressort 27 disposé autour de la portion d'arbre 22 et ayant le même rôle que le ressort 19 de la figure 1.

On se référera maintenant à la figure 2 et aussi, plus spécialement, à la figure 4, lesquelles montrent que le système d'accouplement 21 comporte un arbre creux 28 à l'intérieur duquel l'extrémité amont 20 de l'arbre du mécanisme à pince est capable de coulisser et dont l'extrémité amont est dotée de deux encoches axiales oblongues 29 diamétralement opposées (une seule apparaissant au dessin) ayant le même rôle que les encoches 17 de la figure 1.

La particularité de l'arbre creux 28 est de posséder intérieurement, sensiblement à mi-chemin entre ses extrémités, une paroi 30 formant butée et percée d'une fenêtre rectangulaire 31 conformée et dimensionnée de manière à laisser passer seulement les deux ergots 26 d'extrémité de l'arbre du mécanisme à pince. La largeur de cette fenêtre correspond au côté de la portion amincie 23. La paroi 30 est montrée vue de l'aval sur la figure 4. On notera que le diamètre de la portion amincie 23 au niveau de la saignée 25 correspond à la largeur de la fenêtre 31, afin d'y permettre sa libre rotation.

La figure 2 montre, en a, b et c, les éléments décrits ci-dessus respectivement :
- dans une première position extrême, pour laquelle l'extrémité amont 20 de l'arbre du mécanisme à pince, après avoir traversé la fenêtre 31, est la plus engagée dans l'arbre creux 28 ;
- dans la même position, lorsque l'arbre creux 28 a été tourné de 90°; et
- dans une deuxième position extrême, pour laquelle l'extrémité amont 20 de l'arbre du mécanisme à pince est la moins engagée dans l'arbre creux 28.

La connexion de l'extrémité 20 de l'arbre du mécanisme à pince et du système d'accouplement 21 s'effectue de la manière suivante.

### Figure 2a

L'extrémité 20 de l'arbre du mécanisme à pince est introduite dans l'arbre creux 28, en orientant ces deux pièces par rotation de manière à ce que la portion carrée 23 et les ergots 26 traversent la fenêtre 31. La course des ergots est stoppée lorsque la portion cylindrique 22 vient en butée contre la paroi 30. Le ressort 27 est alors comprimé au maximum. Dans cette position, la saignée circulaire 25 se trouve exactement dans la fenêtre 31 qui la laisse ainsi libre de tourner.

### Figure 2b

L'arbre creux 28 est tourné manuellement de 90°, puisque la saignée 25 l'y autorise.

### Figure 2c

Il ne reste plus, alors, qu'à relâcher l'arbre creux 28 qui, sous l'action de rappel exercée par le ressort 27, va coulisser vers l'aval sur la portion carrée 23 jusqu'à ce que les ergots 26, maintenant décalés de 90°, ne retrouvent plus leur passage à travers la fenêtre 31 et buttent sur la paroi 30. La jonction est alors réalisée.

Les opérations nécessaires à la séparation suivront un chemin inverse et n'ont donc pas à être exposées plus en détail.

Il va de soi que, pour faciliter à l'opérateur la rotation de 90°, des repères visuels peuvent être pratiqués sur le mécanisme à pince et le système d'accouplement.

Ainsi est proposé un instrument à main dans lequel l'assemblage du mécanisme à pince avec le système permettant son accouplement au mécanisme moteur utilise des dispositions faciles à réaliser, offre à l'opérateur une grande facilité d'utilisation et garantit une tenue capable de résister à une connexion alors que le moteur est toujours en rotation. En effet, la saignée 25 pratiquée sur la portion carrée de l'arbre du mécanisme à pince est suffisamment disposée en aval pour qu'au moment de la connexion du mécanisme moteur au système d'accouplement, l'arbre creux 28 de celui-ci ne soit pas repoussé au point que sa fenêtre 31 arrive au niveau de la saignée.

## Revendications

1. Instrument médical à main destiné à faire tourner à grande vitesse un outil amovible, du type comportant un mécanisme à pince pour la fixation de l'outil, un mécanisme moteur pour l'entraînement en rotation du mécanisme à pince et un système d'accouplement (21) du mécanisme à pince avec le mécanisme moteur, ledit système comportant un arbre creux (28) à l'intérieur duquel l'extrémité (20) de l'arbre du mécanisme à pince est capable de coulisser, **caractérisé en ce que** le système d'accouplement (21) et l'extrémité (20) de l'arbre du mécanisme à pince s'assemblent l'un à l'autre au moyen d'une fixation à baïonnette dans laquelle des ergots (26) appartenant à ladite extrémité (20) sont retenus, après rotation, par une butée (30) ménagée à l'intérieur dudit arbre creux (28).

2. Instrument selon la revendication 1, **caractérisé en ce que** l'extrémité (20) de l'arbre du mécanisme à pince comporte une portion cylindrique (22) suivie d'une portion amincie à section carrée (23), une saignée circulaire (25) étant pratiquée dans ladite portion amincie, à l'endroit de sa liaison avec ladite portion cylindrique, et **en ce que** la portion amincie (23) se termine par lesdits ergots (26).

3. Instrument selon la revendication 2, **caractérisé en ce que** ledit arbre creux (28) possède intérieurement une paroi (30) formant ladite butée et percée d'une fenêtre (31) conformée et dimensionnée de manière à laisser passer lesdits ergots (26).

4. Instrument selon la revendication 3, **caractérisé en ce que** le diamètre de ladite portion amincie (23) au niveau de ladite saignée (25) lui permet une libre rotation à l'intérieur de ladite fenêtre (31).
